# EUROPEAN PATENT SPECIFICATION

(11) **EP 2 470 225 B1**
(45) Date of publication and mention of the grant of the patent: **19.03.2014**
(21) Application number: 10749673.9
(22) Date of filing: 26.07.2010
(51) Int. Cl.: A61L 15/32

(54) **COLLAGEN PAD**
KOLLAGENAUFLAGE
COUSSINET DE COLLAGÈNE

(30) Priority: 28.08.2009 GB 0915051
(43) Date of publication of application: 04.07.2012
(73) Proprietor: Tissue Science Laboratories Limited, Fareham, Hampshire P015 7NY (GB)
(72) Inventor: ARMITAGE, Paul, West Yorkshire WF10 2DB (GB); DAWSON, Christine Elizabeth, West Yorkshire WF10 2DB (GB)
(74) Representative: Wilson Gunn
(86) International application number: PCT/GB2010/001411
(87) International publication number: WO 2011/023926

(56) References cited:
- EP-A1- 0 913 162
- WO-A1-00/15274
- GB-A- 963 772
- US-A- 5 749 895

## Description

The present invention relates to a collagen pad and to a process for the manufacture thereof. The collagen pad may be used, for example, in wound care.

Collagen is a triple-helix protein which forms the major part of the dermal extracellular matrix (ECM), together with glycosaminoglycans, proteoglycans, laminin, fibronectin, elastin and cellular components. The ECM is the largest component of the dermal skin layer and synthesis of ECM is a key feature of wound healing, especially when there has been a significant loss of tissue that precludes closure by primary intention.

The principal function of collagen in the dermal ECM is to act as a scaffold in connective tissue. Predominantly, collagen is present in the form of type I collagen (80-85%) and type III collagen (8-11%), both of which are fibrillar or rod-shaped collagens. The tensile strength of skin is due largely to these collagen molecules assembling into fibrils, with adjacent molecules cross-linking to further increase tensile strength. However, collagen is laid down during wound healing has a reduced structural integrity compared to unwounded tissue; scar tissue rarely exceeds 70% of unwounded tissue strength.

In addition to being the main component of scar tissue, collagen has a key role in the control of inflammatory responses to injury and subsequent repair with functions that influence cellular mitogenesis, differentiation and migration, protein synthesis in the ECM, synthesis and release of inflammatory cytokines and growth factors, and interactions between enzymes which remodel the ECM, including matrix metalloproteinases (MMPs) and their tissue inhibitors (TIMPs).

For medical applications, purified fibrous collagen has long been known to be a useful therapeutic adjunct to aid wound healing. Research into collagen-based dressing materials has shown that collagen significantly increases the production of fibroblasts in wounds, encouraging direct migration of cells into the wound enhancing the formation of new, organised collagen fibres.

Purified fibrous collagen pads have also been used to provide haemostatic dressings. When in contact with a bleeding surface, a fibrous collagen haemostat attracts platelets which adhere to collagen fibrils and release coagulation factors. This coagulation and aggregation of the platelets into thrombi on the collagenous mass, provides the formation of a physiologic platelet plug which slows and eventually stops the bleeding. The collagen fibrils also provide a structural matrix strengthening the platelet plug.

The document US57499895 discloses a method for the manufacture of a collagen pad comprising the steps of forming a solution of collagen and precipitating the collagen by addition of sodium phosphate.

Previous studies have demonstrated that collagenous materials prepared using a process that retains the original fibre architecture and molecular ultrastructure of the natural tissues from which they are derived have a number of advantageous properties. For example, US 5397353 discloses a process for the preparation of collagenous materials that are substantially non-antigenic and substantially free of non-fibrous tissue proteins, cellular elements, lipids and lipid residues. The materials are typically sheet structures and are useful, for example, as implants. The collagenous sheet materials are susceptible to colonisation and vascularisation by host cells following implantation and are resistant to calcification.

It has further been shown that the favourable properties of these collagen sheet materials can be retained when the collagen material is presented in the form of particles comprising fibre fragments. EP 1112096 describes an injectable or mouldable composition of collagen fibre fragments prepared from the relatively large-scale sheet materials of US 5397353 in such a way as to retain the natural collagen fibre architecture and molecular ultrastructure. This size reduction is achieved by careful grinding or milling of the collagenous sheet materials, which may then be suspended to form a paste or injectable composition, as appropriate.

The present invention provides a novel collagenous material particularly suitable for use as a wound dressing.

According to the present invention, there is provided a process for the manufacture of a collagen pad from a plurality of collagen particles, said process comprising steps of:
(i) treating a natural tissue material with an organic solvent;
(ii) treating the natural tissue material with a proteolytic enzyme, so as to provide a treated material that is substantially free of non-fibrous tissue proteins, cellular elements and lipids or lipid residues;
(iii) forming a plurality of collagen particles from the treated material, said collagen particles comprising fragments of collagen fibres displaying the original collagen fibre architecture and molecular ultrastracture of said natural tissue material;
(iv) forming a dispersion of the collagen particles in an aqueous acid solution
(v) adding a flocculating agent to the dispersion to form a collagen floc; and
(vi) applying a compressive force to the collagen floc.

The resulting collagen floc may be separated from the aqueous phase and recovered for use as a collagen pad. The collagen floc forms upon the surface of the aqueous phase, and recovery is therefore relatively straightforward. The cohesive collagen material is relatively light and has a relatively open structure, being similar to cotton wool in appearance.

A force is applied to the collagen floc in order to increase the density of the resulting collagen pad. Thus, the process additionally comprises a step of applying a compressive force to the collagen floc to produce a collagen pad.

In a preferred embodiment, force is applied to the floc by means of centrifugation. Thus, the product of the flocculation reaction, i.e. the reaction comprising the adding of the flocculating agent to the dispersion of collagen particles in aqueous acid solution to form the collagen floc, may be subjected to centrifugation. This step of centrifuging facilitates separation and recovery of the resulting collagen pad from the aqueous phase. Surprisingly, the centrifuged collagen pad remains upon the surface of the aqueous phase, such that recovery is relatively simple. The forces applied to the collagen floc during the centrifugation step result in compression of the floc to form a collagen pad of increased density. The collagen pad has good structural integrity.

The centrifugation step may be carried out using any suitable centrifugation apparatus. The centrifugation conditions can be varied to produce collagen pads of varying densities. At lower forces and/or centrifugation times, a less dense collagen pad is formed, whereas increasing the centrifugal force and/or centrifugation time results in greater compression of the collagen pad.

It will be appreciated, therefore, that a wide range of centrifugation conditions may be employed. For example, centrifugation may be carried out at about 5 g to about 6000 g or more, and the centrifugation time may vary from around 5 minutes to around 30 minutes or more. Good results have been achieved by centrifuging at about 5250 g for about 5 minutes.

According to a further aspect of the present invention, there is provided a process for the manufacture of a collagen pad, wherein said process comprises a step of centrifuging the product of a flocculation reaction, said flocculation reaction comprising adding a flocculating agent to a dispersion of collagen particles in an aqueous acid solution to form a collagen floc.

Any suitable aqueous acid solution may be used to form the dispersion of collagen particles. The choice of acid employed is not critical since the purpose is to lower the pH so that individual collagen particles readily absorb water. A weak organic or inorganic acid is generally used. Non-limiting examples include dilute hydrochloric acid, acetic acid, and boric acid. Particularly good results have been observed using glacial acetic acid.

The concentration of the acid is typically at least 0.1% in order to provide a suitable pH for swelling. Acid can have a detrimental effect on the structure of collagen and therefore care should be taken to ensure that the aqueous acid solution is not too concentrated. The acid concentration should generally not exceed 1%. Typically, the pH is between 1 and 4, and more typically is between 2 and 4. Particularly good results have been achieved at around pH 3 to 3.5.

In preferred embodiments, the collagen particles are suspended in the aqueous acid solution and mixed to increase the rate and extent of dispersion. The collagen particles may be weighed to determine the amount of aqueous acid solution required. Typically, solid collagen content may vary from about 1% to about 5% of the aqueous acid solution volume. For example, good results have been observed using a formulation of about 1% collagen in aqueous acid solution.

Mixing can be achieved by various means, including mixing by hand or using mechanical mixing apparatus. For example, mixing can be carried out by homogenising, agitating, shaking, or blending. Preferably, the collagen particles are dispersed in the aqueous acid solution by homogenisation.

Homogenisation of the suspension of swollen collagen particles results in a dispersion in the form of a slurry or 'paste' of swollen collagen particles. If fully dispersed, the combination of swollen collagen particles and aqueous acid solution may be considered a colloid.

Typically, solid collagen content may vary from about 1% to about 5% of the solution volume. As a consequence of the reduced pH, the collagen particles swell in the aqueous acid solution and the increase in particle size reduces the fluidity of the dispersion. At concentrations of, for example, around 1% the dispersion can be easily mixed. However, higher concentrations of collagen become difficult to mix due to the considerable increase in viscosity. At concentrations of about 2% and above, the dispersion may be relatively difficult to mix.

Flocculation is defined by the International Union of Pure and Applied Chemistry as a process of contact and adhesion whereby dispersed particles are held together by weak physical interactions ultimately leading to phase separation by the formation of precipitates of larger than colloidal size.

In the present invention, any suitable flocculating agent may be used to achieve flocculation. The flocculating agent may be added gradually, in a step-wise manner.

Preferred flocculating agents include bases, in particular alkalis. The pH of the collagen dispersion is increased by a basic flocculating agent. It is thought that this neutralises the particulate surface change within the dispersion, thereby destabilising the dispersion and allowing the collagen particles to aggregate.

Conveniently, an aqueous alkali solution may be used here as the flocculating agent, for instance sodium hydroxide solution. By way of example, good results have been observed using sodium hydroxide at a concentration of around 2M, although a range of different aqueous alkali solutions may be used as flocculating agents.

The pH is preferably increased to at least pH 4.5. It has been found that below around pH 4.5 the flocculation reaction may not be initiated or may be incomplete. The pH may be increased to within the range of 4.5 to 12. It is generally preferred to operate at a pH towards the lower end of this range, such as pH 4.5 to 9, to minimise the likelihood of structural damage to the collagen and to reduce the quantity of basic flocculating agent required. As the pH is increased to the higher end of the range, the resulting collagen floc may have reduced structural integrity.

To facilitate flocculation, it may be desirable to mix the dispersion during the addition of the flocculating agent. Mixing can be achieved by any means, including mixing by hand or using mechanical mixing apparatus. For example, mixing can be carried out by homogenising, agitating, shaking, or blending.

Small collagen aggregates formed during flocculation rise to the surface of the aqueous phase and join together to form a cohesive collagen mass, or floc.

Optionally, the collagen pad may be dehydrated. Thus, the process may comprise an additional step of dehydrating the collagen pad. Dehydration helps to reduce potential bioburden growth.

Usefully, in embodiments comprising a centrifugation step the collagen pad is typically already dehydrated to some extent by the action of the centrifuge which forces some of the aqueous solution from the collagen pad.

In certain embodiments, dehydration is carried out by treatment of the collagen pad with a dehydrating agent. Any suitable dehydrating agent may be used. For example, the dehydrating agent may comprise any water miscible solvent that does not react with or dissolve collagen. Non-limiting examples of suitable dehydrating agents include non-aqueous solvents such as acetone, ethanol, ether, or mixtures thereof. The volume of solvent required to dehydrate the collagen pad is dependant upon the specific solvent(s) used and volume of collagen mass present. By way of example, acetone may be used at a ratio of about 5:1 solvent to collagen by volume. Multiple solvent rinses may be required to ensure complete removal of water. For instance, good results have been achieved using 3 x 45-minute rinses with acetone.

In alternative embodiments, the collagen pad may be dehydrated by air drying.

Optionally, cross-linking may be carried out to impart additional physical strength to the collagen pad, and an increased resistance to digestive enzymes that may be present in a wound healing environment.

Typically, cross-linking is carried out post-centrifugation and preferably after dehydration of the collagen pad. Additionally or alternatively, cross-linking may be carried out following flocculation.

Whilst any cross-linking agent may be used, preferred cross-linking agents include polyisocyanates, in particular diisocyanates. The polyfunctional isocyanates react with amino or hydroxyl groups of different protein chains, improving stability and resistance to enzymatic attack. It is known that antigenicity is associated with the amino groups of the protein chains of collagen, and reacting the amino groups with isocyanate removes any antigenicity associated with these groups. Preferred diisocyantes include aliphatic, aromatic and alicyclic diisocyanates as exemplified by 1,6-hexamethylene diisocyanate, toluene diisocyanate, 4,4'-diphenylmethane diisocyanate, and 4,4'-dicyclohexylmethane diisocyanate, respectively. A Particularly preferred diisocyanate is hexamethylene diisocyanate (HMDI). Carbodiimide cross-linking agents may also be used, such as 1-ethyl-3-[3-dimethylaminopropyl]carbodiimide hydrochloride (EDC).

Where the cross-linking agent is used to treat non-dehydrated collagen material, a surfactant may be used to ensure proper dispersion of the cross-linking agent.

The extent of cross-linking may be varied. Usefully, this provides a mechanism for controlling the rate at which the collagen pad is resorbed or degraded during use. The resistance to degradation tends to increase as the extent of cross-linking is increased.

By way of example, cross-linking may be carried out using HMDI. As a guide, the HMDI may be used at a concentration of around 0.01g to 0.5g per 50g of collagen. Cross-linking may be carried out for a range of different time periods. By way of example, the collagen may be exposed to the cross-linking agent for between around 1 hour and around 3 days. Typically, cross-linking is carried out for at least 12 hours, preferably at least 20 hours.

It will be appreciated that the cross-linking conditions may routinely be varied in order to adjust the extent of cross-linking.

The collagen pad described herein has excellent properties for use as a wound dressing or as a component of a wound dressing. This porous, biocompatible material is non-antigenic and of natural origin, and provides a skin/tissue-like feel which improves patient acceptance and satisfaction. The collagen pad is soft and conformable, which is clearly advantageous for application to wound sites. Significantly, the collagen pad has good inherent strength and may be wrapped around wound areas to form an effective barrier. The collagen pad maintains a moist wound environment to promote wound healing, and its application helps to reduce contraction and scar tissue formation. Unlike a number of existing materials used in wound dressings, the collagen pad does not gel on wound contact.

Thus, collagen pad material described herein is particularly useful in wound care. The collagen pad may be presented in a hydrated state. Thus, the process may further comprise at least one hydration step, in which the collagen pad is maintained in an aqueous solution. For instance, the collagen pad may be maintained in saline, such as 0.9% saline. The presentation of the collagen pad in a hydrated form helps to maintain a moist wound environment when the material is applied as a wound dressing.

The collagen particles may be derived from any collagen-containing natural tissue material of human or animal origin. The natural tissue material may be a tissue comprising predominantly type I collagen. Preferred starting materials include dermis and tendons. In some embodiments, it is preferred that porcine tissue materials are processed to provide the collagen pad, although it will be understood that other mammalian sources may alternatively be employed, such as, for example, primates, cows, sheep, goats or horses.

Preferably, the collagen particles are substantially free of non-fibrous tissue proteins, cellular elements and lipids or lipid residues. Depending upon the starting material, the particles of collagen may contain a proportion of elastin. Thus, the particles, and the collagen pad formed therefrom, consist essentially of collagen optionally with small proportions of elastin.

The collagen particles may be of any suitable size. Typically, the collagen particles have a mean diameter within the range of from around 5µm to around 1000µm, more typically from around 50µm to around 500µm. Good results have been achieved using collagen particles with a mean diameter of approximately 150µm.

The particles of collagen may be formed using any suitable process.

In preferred embodiments, the collagen particles display original collagen fibre architecture and molecular ultrastructure of the natural tissue material from which they are derived. Preferred processes for preparing the collagen particles are analogous to those described in EP 1112096. Preferably, collagenous material prepared in the form of a sheet or other large-scale structure is milled to form the particles. The collagenous material may be prepared by a process analogous to those described in US 5397353. The collagenous tissue is neither solubilised nor denatured in the process, so its natural structure is maintained.

Thus, freshly cut natural tissue material may be treated to remove therefrom substantially all lipids and lipid residues and thereafter treated to remove non-fibrous tissue proteins and cellular elements.

The lipid extraction may be achieved by solvent extraction using an organic solvent, such as acetone. Other non-limiting examples of suitable solvents include non-aqueous solvents such as ethanol and ether.

Non-fibrous tissue proteins include glycoproteins, proteoglycans, globular proteins and the like. Cellular elements include antigenic proteins and enzymes and other cellular debris arising from the processing conditions. These portions of the natural tissue material may be removed by treatment with a proteolytic enzyme, such as trypsin. It has previously been found that above 20°C treatment with trypsin can in some circumstances result in an alteration of the collagen fibre structure leading to a lower physical strength. Moreover, low temperatures discourage the growth of microorganisms in the preparation. It is therefore preferred to carry out the treatment with trypsin at a temperature below 20°C. Moreover, trypsin is more stable below 20°C and lower amounts of it may be required. Any suitable trypsin concentration may be used, for instance a concentration within the range of around 0.01g/l to 25g/l. It has been found that good results can be obtained using about 2.5g/l trypsin.

In the present invention, the natural tissue material is treated with a solvent, preferably acetone, and a proteolytic enzyme, preferably trypsin.

Further treatments may optionally be carried out, such as treatment with one or more additional enzymes, for example a carbohydrate-splitting enzyme.

Those substances said to be "substantially free" of materials generally contain less than 5% of, and preferably less than 1%, of said materials.

The resulting collagenous material is then reduced to particles, care being taken to ensure that the size reduction is not associated with a degradation of the original collagen fibre architecture and molecular ultrastructure of the starting material. The particles may be produced by grinding or milling using, for example, a ball or hammer mill, which may be cooled to an appropriate temperature. The sheet material may be cut into small pieces prior to milling. Milling may be carried out in dry form (less than 10% moisture content) or in frozen hydrated form (20-80% moisture content).

Scanning electron microscopy (SEM) analysis of collagen pads manufactured in accordance with these preferred embodiments has revealed that the constituent collagen fibrils appear generally to maintain the alignment seen in fragments of collagen fibres and fibre bundles making up the collagen particles.

According to a further aspect of the present invention there is provided a collagen pad obtainable by a process as herein described.

A range of different factors may be added to the collagen pad, such as growth factors, clotting agents, or other pharmaceutically active agents. The collagen pad may be seeded with cells, such as stem cells.

The collagen pad is useful in wound care. The collagen pad may also be used as a haemostat, to reduce or prevent blood flow from a body site.

According to a further aspect of the present invention there is provided a collagen pad as herein described for use in therapy.

According to a further aspect of the present invention there is provided the use in therapy of a collagen pad as herein described.

According to a further aspect of the present invention there is provided a collagen pad as herein described for use in wound care.

According to a further aspect of the present invention there is provided the use in wound care of a collagen pad as herein described.

According to a further aspect of the present invention there is provided a method of treatment of a wound comprising the step of applying to the wound a collagen pad as herein described.

According to a further aspect of the present invention there is provided a collagen pad as herein described for use as a haemostat.

According to a further aspect of the present invention there is provided the use in haemostasis of a collagen pad as herein described.

According to a further aspect of the present invention there is provided a method of reducing bleeding from a body site comprising the step of applying to the body site a collagen pad as herein described.

Embodiments of the present invention will now be described further in the following non-limiting examples and with reference to the accompanying drawings, in which:
- Fig. 1: is a scanning electron micrograph at x1000 magnification of a representative collagen pad according to the present invention;
- Fig. 2: is a scanning electron micrograph at x5000 magnification of the collagen pad of Fig. 1; and
- Fig. 3: is a scanning electron micrograph at x10000 magnification of the collagen pad of Figs. 1 and 2;

### Examples

### 1. Manufacture of collagen pad

Acellular collagen sheet material was prepared according to the method disclosed in US 5397353 and reduced to particles as described in EP 1112096.

Thus, freshly cut dermis harvested from sows was immersed in acetone. After 1 hour, the acetone was removed and replaced by fresh acetone. After a further incubation for around 36 hours, the tissue was removed from the acetone and placed in 0.9% saline to extract residual acetone. The tissue was then digested for 28 days with a solution of trypsin at a concentration of 2.5 mg/ml in 0.1M phosphate buffer with 0.5 mg/ml sodium azide as a bacteriostatic agent. The purified tissue was removed from the trypsin solution and rinsed in buffer.

The resulting collagenous sheet material was cut into small pieces (approximately 5mm x 10mm in size) before being milled cryogenically to a mean particle size of around 150µm.

The collagen particles were suspended in a 0.6% glacial acetic acid solution such that a concentration of about 1% solid collagen was formulated. This suspension was homogenised for about 30 seconds using a Silverson^{®} L4RT homogeniser to evenly disperse the swollen collagen particles throughout the suspension, thereby forming a slurry or 'paste' of swollen collagen particles.

With continuous mixing, a pH probe was introduced to the collagen dispersion and 2M sodium hydroxide solution was added drop-wise until such a time that a pH of around 9 was recorded. At this point, the pH probe was removed and the dispersion homogenised continuously for an additional 90 seconds. A collagen floc was observed on the surface of the aqueous phase. This cohesive collagen material had a relatively open structure, being similar to cotton wool in appearance.

The entire reaction mixture was then transferred to a centrifuge container and centrifuged at a speed of about 5250 x g (about 4750 rpm) for 5 minutes.

The centrifuged solid collagen material remained upon the surface of the aqueous phase, such that recovery was straightforward. The forces applied to the collagen floc during the centrifugation step compressed the floc to form a collagen pad of increased density. The collagen pad had good structural integrity.

The collagen pad was removed from the centrifuge container and rinsed in acetone. Cross-linking of the collagen was then carried out using HMDI in acetone at a concentration of about 0.1ml HMDI per 50g of collagen, for a period of 20 hours.

After cross-linking, the collagen pad was rinsed in 0.9% saline to remove residual chemicals and after final rinsing stored in saline. The pad was then gamma irradiated at a dose of ≥25kGys.

### 2. SEM analysis

The collagen pad of Example 1 was examined by SEM. Samples of approximately 7mm x 7mm were cut from collagen pads and mounted on SEM stubs using double-sided sticky tabs. Silver-dag was used around the edges to reduce charge effects. The samples were sputter coated with approximately 5nm of gold/palladium. They were viewed at 2kV using a JEOL JSM-7500F scanning electron microscope at a working distance of approximately 8mm and the LEI detector.

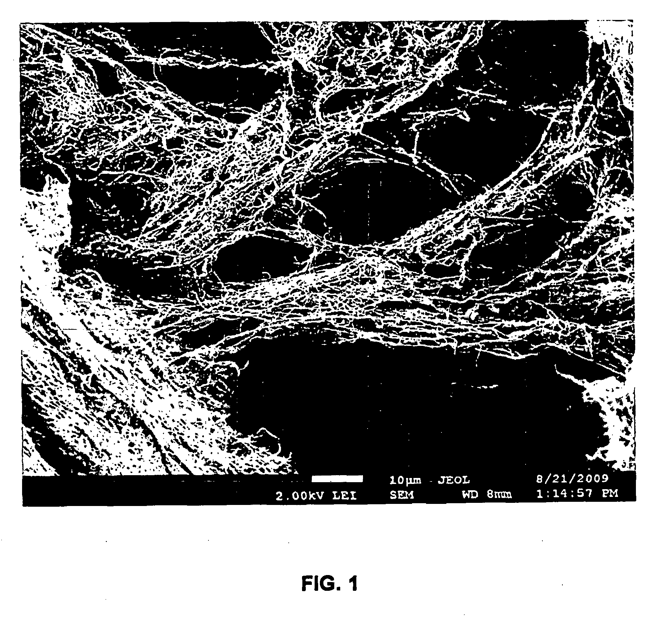

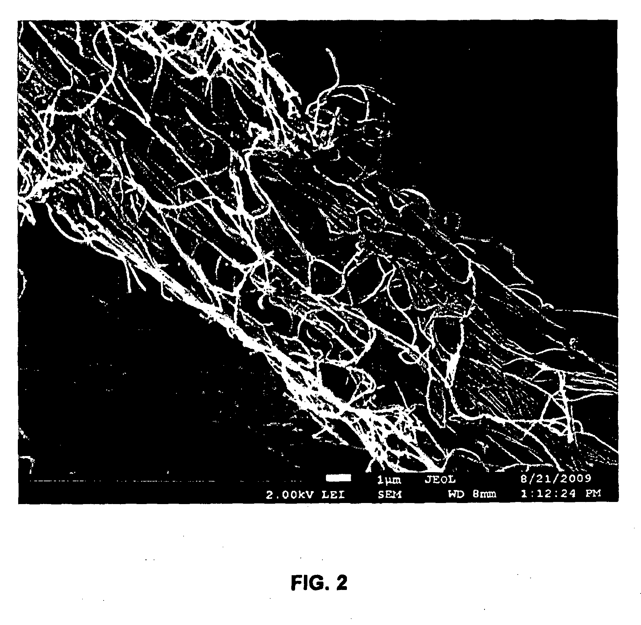

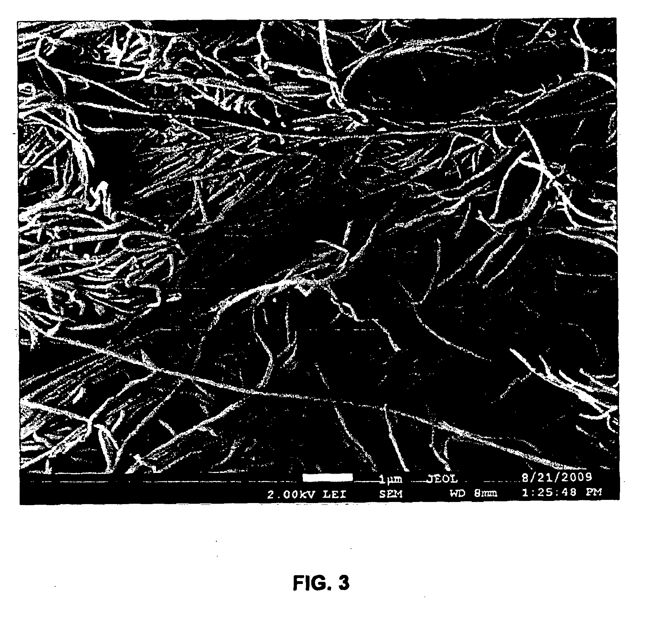

Representative results are shown in Fig. 1 (x1000 magnification), Fig. 2 (x5000 magnification) and Fig. 3 (x10000 magnification). Referring to the scanning electron micrograph of Fig. 1, it can be seen that the collagen pad has an open structure. At the higher magnifications of Figs. 2 and 3, the intact collagen fibrils can be observed. The collagen fibrils appear generally aligned in the manner of the collagen fibres and fibre bundles making up the collagen starting materials.

### 3. Tensile strength testing

The tensile strength of the collagen pad of Example 1 was tested using a Hounsfield tensiometer equipped with a 1kN load cell. Jaw speed was set at 10mm/min, with samples tested until failure. For reference, testing was repeated using samples of Promogran®, an existing wound care product comprising a freeze-dried composite of oxidised regenerated cellulose and collagen.

Testing was carried out using representative 100mm x 10mm samples, fully hydrated in 0.9% saline. Maximum load before failure was recorded to provide ultimate tensile strength in Newtons.

The collagen pad of Example 1 was found to have a tensile strength of around 2.0 N in the hydrated state. The hydrated Promogran® material had a tensile strength of around 0.1 N.

## Claims

1. A process for the manufacture of a collagen pad, comprising the steps of:
(i) treating a natural tissue material with an organic solvent;
(ii) treating the natural tissue material with a proteolytic enzyme,
so as to provide a treated material that is substantially free of non-fibrous tissue proteins, cellular elements and lipids or lipid residues;
(iii) forming a plurality of collagen particles from the treated material, said collagen particles comprising fragments of collagen fibres displaying the original collagen fibre architecture and molecular ultrastracture of said natural tissue material;
(iv) forming a dispersion of the collagen particles in an aqueous acid solution;
(v) adding a flocculating agent to the dispersion to form a collagen floc; and
(vi) applying a compressive force to the collagen floc.

2. A process according to claim 1, wherein the proteolytic enzyme comprises trypsin.

3. A process according to claim 1 or claim 2, wherein the compressive force is applied to the collagen floc by centrifugation.

4. A process according to any one of the preceding claims, wherein the aqueous acid solution has a pH of 3 to 3.5.

5. A process according to any one of the preceding claims, wherein the collagen particles are dispersed in the aqueous acid solution by homogenisation.

6. A process according to any one of the preceding claims, wherein the flocculating agent is an aqueous alkali solution.

7. A process according to claim 6, wherein the pH is increased to at least 4.5 by the addition of the aqueous alkali solution.

8. A process according to claim 6 or claim 7, wherein the pH is increased to within the range 4.5 to 9 by the addition of the aqueous alkali solution.

9. A process according to any one of the preceding claims, wherein the process comprises a step of dehydrating the collagen pad.

10. A process according to any one of the preceding claims, wherein the process comprises a step of cross-linking the collagen.

11. A collagen pad obtainable by a process according to any one of the preceding claims.

12. A collagen pad according to claim 11 for use in therapy.

13. A collagen pad according to claim 11 or claim 12 for use in wound care.

14. A collagen pad according to claim 11 or claim 12 for use as a haemostat.

## Patentansprüche

1. Verfahren zur Herstellung eines Kollagenpads, das die Schritte umfasst:
(i) Behandeln eines natürlichen Gewebematerials mit einem organischen Lösungsmittel,
(ii) Behandeln des natürlichen Gewebematerials mit einem proteolytischen Enzym, um ein behandeltes Material bereitzustellen, das im Wesentlichen frei von nicht-faserigen Gewebeproteinen, zellulären Elementen und Lipiden oder Lipidresten ist,
(iii) Bilden einer Mehrzahl von Kollagenteilchen aus dem behandelten Material, wobei diese Kollagenteilchen Bruchstücke von Kollagenfasern aufweisen, die den ursprünglichen Aufbau der Kollagenfasern und die molekulare Ultrastruktur des natürlichen Gewebematerials zeigen,
(iv) Bilden einer Dispersion aus den Kollagenteilchen in einer wässrigen, sauren Lösung,
(v) Zugeben eines Flockungsmittels zu der Dispersion zur Bildung von Kollagenflocken, und
(vi) Aufbringen einer Druckkraft auf die Kollagenflocken.

2. Verfahren nach Anspruch 1, wobei das proteolytische Enzym Trypsin aufweist.

3. Verfahren nach Anspruch 1 oder Anspruch 2, wobei die Druckkraft mittels Zentrifugieren auf die Kollagenflocken aufgebracht wird.

4. Verfahren nach einem der vorherigen Ansprüche, wobei die wässrige, saure Lösung einen pH-Wert von 3 bis 3,5 aufweist.

5. Verfahren nach einem der vorherigen Ansprüche, wobei die Kollagenteilchen durch Homogenisieren in der wässrigen, sauren Lösung dispergiert werden.

6. Verfahren nach einem der vorherigen Ansprüche, wobei es sich bei dem Flockungsmittel um eine wässrige, alkalische Lösung handelt.

7. Verfahren nach Anspruch 6, wobei der pH-Wert durch die Zugabe der wässrigen, alkalischen Lösung auf mindestens 4,5 erhöht wird.

8. Verfahren nach Anspruch 6 oder Anspruch 7, wobei der pH-Wert durch die Zugabe der wässrigen, alkalischen Lösung auf einen Wert im Bereich von 4,5 bis 9 erhöht wird.

9. Verfahren nach einem der vorherigen Ansprüche, wobei das Verfahren einen Schritt des Dehydratisierens des Kollagenpads umfasst.

10. Verfahren nach einem der vorherigen Ansprüche, wobei das Verfahren einen Schritt des Vernetzens des Kollagenpads umfasst.

11. Kollagenpad, das durch ein Verfahren nach einem der vorherigen Ansprüche erhalten werden kann.

12. Kollagenpad nach Anspruch 11 zur Anwendung bei der Therapie.

13. Kollagenpad nach Anspruch 11 oder Anspruch 12 zur Anwendung bei der Wundpflege.

14. Kollagenpad nach Anspruch 11 oder Anspruch 12 zur Anwendung als Hämostasemittel.

## Revendications

1. Procédé de fabrication d'un coussinet de collagène comprenant les étapes suivantes :
(i) traiter un tissu naturel au moyen d'un solvant organique ;
(ii) traiter le tissu naturel au moyen d'une enzyme proteolytique ; afin d'obtenir un matériau traité qui est substantiellement dénué de protéines non fibreuses de tissu, d'éléments cellulaires et de lipides ou résidus de lipides ;
(iii) former une pluralité de particules de collagène à partir du matériau traité, lesdites particules de collagène comportant des fragments de fibres de collagène présentant l'architecture de la fibre de collagène originale et l'ultrastructure moléculaire dudit matériau de tissu naturel
(iv) former une dispersion des particules de collagène dans une solution aqueuse acide ;
(v) ajouter un agent floculant à la dispersion de façon à réaliser un floc ; et
(vi) appliquer une force de compression au floc de collagène.

2. Procédé selon la revendication 1, dans lequel l'enzyme proteolytique comporte de la trypsine.

3. Procédé selon la revendication 1 ou 2, dans lequel la force de compression est appliquée au collagène par centrifugation.

4. Procédé selon l'une quelconque des revendications précédentes, dans lequel la solution aqueuse acide a un pH de 3 à 3,5.

5. Procédé selon l'une quelconque des revendications précédentes, dans lequel les particules de collagène sont dispersées dans la solution aqueuse acide par homogénisation.

6. Procédé selon l'une quelconque des revendications précédentes, dans lequel l'agent floculant est une solution aqueuse alcaline.

7. Procédé selon la revendication 6 dans lequel le pH est augmenté jusqu'à au moins 4,5 par addition de la solution aqueuse alcaline.

8. Procédé selon la revendication 6 ou 7, dans lequel le pH est augmenté jusqu'à une gamme de 4,5 à 9 par addition de la solution aqueuse alcaline

9. Procédé selon l'une quelconque des revendications précédentes, dans lequel le procédé comporte l'étape de déshydrater le coussinet de collagène.

10. Procédé selon l'une quelconque des revendications précédentes, dans lequel le procédé comprend l'étape de réticulation du collagène.

11. Coussinet de collagène obtenu par le procédé selon l'une quelconque des revendications précédentes.

12. Coussinet de collagène selon la revendication 11 à usage thérapeutique.

13. Coussinet de collagène selon la revendication 11 ou 12 à usage pour le soin de blessure.

14. Coussinet de collagène selon la revendication 11 ou 12 à usage hémostatique.
